**Europäisches Patentamt**

**European Patent Office**

**Office européen des brevets**

(11) Veröffentlichungsnummer: **0 326 143**
**A1**

## (12) EUROPÄISCHE PATENTANMELDUNG

(21) Anmeldenummer: 89101388.0

(22) Anmeldetag: 27.01.89

(51) Int. Cl.4: **A61K 41/00 , A61K 7/00 , A61K 9/00**

(30) Priorität: **28.01.88 DE 3802521**

(43) Veröffentlichungstag der Anmeldung:
**02.08.89 Patentblatt 89/31**

(84) Benannte Vertragsstaaten:
**AT CH DE ES FR GB IT LI**

(71) Anmelder: **Müller, Bernhard**
**Herdweg 101**
**D-7000 Stuttgart 1(DE)**

(72) Erfinder: **Müller, Bernhard**
**Herdweg 101**
**D-7000 Stuttgart 1(DE)**

(74) Vertreter: **Nöth, Heinz, Dipl.-Phys. et al**
**Patentanwälte Pfenning, Meinig & Partner**
**Mozartstrasse 17**
**D-8000 München 2(DE)**

(54) **Verfahren zur Behandlung von Substanzen in einem Magnetfeld.**

(57) Verfahren zur Behandlung von Substanzen, aus denen Kosmetika, Heilmittel-Extrakte oder äußerlich anzuwendende Arzneimittel hergestellt werden, bei dem die Substanzen während einer bestimmten Verweilzeit dem Einfluß eines Magnetfeldes, das ein statisches Feld oder Wechselfeld sein kann, ausgesetzt werden.

EP 0 326 143 A1

## Verfahren zur Behandlung von Substanzen in einem Magnetfeld

Die Erfindung betrifft ein Verfahren zur Behandlung von Substanzen, aus denen Kosmetika oder Heilmittel-Extrakte (nach einem Extraktionsverfahren gewonnene Arzneimittel) oder äußerlich anzuwendende Arzneimittel hergestellt werden.

Bei Kosmetika handelt es sich um Stoffe oder Zubereitungen aus Stoffen, die zur Reinigung, Pflege oder zur Beeinflussung des Aussehen des menschlichen Körpers oder Körperteilen sowie zur Vermittlung von Geruchseindrücken angewendet werden. Diese Stoffe kommen in Form von Salben, Ölen, Cremes, Liquida, Gelen, Seifen und dgl. in den Handel.

Unter Heilmittel-Extrakten versteht man Stoffe, die aus geeigneten Drogen, tierischen Zellen oder tierischen Organen, unter Zuhilfenahme von Extraktionsmitteln, insbesondere Wasser, Isopropanol, Ethanol oder deren Gemische mit Wasser, durch Extraktion und teilweises oder völliges Eindampfen der Extraktionslösung gewonnen werden. Es kann sich hier um Tinkturen, Trocken-, Fluid- oder Dickextrakte handeln.

Bei äußerlich anzuwendenden Arzneimitteln handelt es sich im wesentlichen um solche, die perkutan, z. B. in Form von Salben, Linimenten, Pasten, Emulsionen oder auch Pflastern zur Anwendung gebracht werden.

Aufgabe der Erfindung ist es, ein neues Behandlungsverfahren für Substanzen, aus denen Kosmetika, Heilmittel-Extrakte oder äußerlich anzuwendende Arzneimittel hergestellt werden, zu schaffen, das insbesondere auf die Struktur dieser Stoffe einwirkt und diese beeinflußt.

Diese Aufgabe wird erfindungsgemäß dadurch gelöst, daß die Substanzen, aus denen die Kosmetika hergestellt werden, während einer bestimmten Verweilzeit dem Einfluß eines Magnetfeldes, das ein statisches Feld oder ein Wechselfeld sein kann, ausgesetzt werden. Die Substanzen können dabei während des Herstellungsverfahrens eines Produktes oder auch am gefertigten Produkt dem Einfluß des Magnetfeldes ausgesetzt werden.

Die Verweilzeit liegt je nach zu behandelnder Substanz normalerweise in der Größenordnung von Minuten bis zu mehreren Stunden. In Abhängigkeit von der Chargengröße bzw. deren Durchmesser und der Stärke des eingesetzten Magnetfeldes kann diese aber auch wesentlich kürzer oder länger sein. Die Verweilzeit kann in Ausnahmefällen auch einige Tage dauern. Die Flußdichten, welche das Magnetfeld aufweisen soll, liegen etwa im Bereich von $10^{-2}$ bis einigen (etwa 3) Tesla.

Zum Aufbau des Magnetfeldes kann ein Permanentmagnet verwendet werden, der die entsprechende magnetische Flußdichte hat.

Für die Permanentmagnete kommen harte magnetische Werkstoffe bevorzugt zur Anwendung, insbesondere Legierungen von Eisen mit Aluminium, Nickel, Kobalt, Kupfer. Es sind jedoch auch eisenfreie Legierungen, z. B. Heuslersche Legierungen, als Dauermagneten verwendbar. Ferner eignen sich Seltenerdmetall-Kobaltlegierungen.

Der bzw. die zur Anwendung kommenden Permanentmagnete können als Ringmagnete oder zylinderförmige Magnete mit an den Endflächen liegenden Polen ausgebildet sein. Die Feldlinien fließen dann durch das Ring- bzw. Zylinderinnere, in welchem auch der Behandlungsraum für die zu behandelnden Substanzen liegt.

Der Ringmagnet bzw. zylinderförmige Magnet kann jedoch auch einen Luftspalt aufweisen, wobei die Pole an den aufeinander zu gerichteten Begrenzungsflächen des Luftspaltes liegen.

Im Luftspalt dieses Permanentmagneten oder im Luftspalt zweier Permanentmagnete, die mit unterschiedlichen Polen aufeinander zu gerichtet sind, befindet sich die zu behandelnde Substanz. Es kann auch ein Elektromagnet zur Anwendung kommen, in dessen Luftspalt die Substanzen behandelt werden.

Es können auch mehrere hintereinander angeordnete Magnete vorgesehen sein, in deren Luftspalten bzw. Behandlungsräumen, beispielsweise in einem Durchlaufverfahren, die Substanzen behandelt werden.

Die Substanzen können auch in einem Kreislauf geführt werden und wiederholt durch das bzw. die Magnetfelder geführt werden. Natürlich eignet sich auch ein Bad, in welchem die gegebenenfalls umgerührte Substanz während der gewünschten Verweilzeit in das Magnetfeld gebracht ist. Vor allem bei der Verwendung von elektromagnetischen Wellenfeldern eignet sich eine Badanordnung, da hier eine einfache Ankoppelung der zu behandelnden Substanz an einen oder mehrere Schwingkreise erreicht werden kann.

Bei der Verwendung von elektromagnetischen Wellenfeldern, die von einem an die zu behandelnde Substanz angekoppelten Schwingkreis abgegeben werden, können diese im Langwellen-bis Mikrowellenbereich liegen.

Die Erfindung kann sowohl im Produktionsverfahren bei den Mischungen, aus denen die Kosmetika bzw. Heil- und Arznei-mittel hergestellt werden, als auch bei den fertigen Produkten zur Anwendung kommen. Hierzu kann ein Permanentmagnet bzw. Permanentmagnet-Werkstoff in Form eines dünnen

2

Plättchens oder als Folie oder als Beschichtung an der Ver packung bzw. am Behälter für das Produkt vorgesehen sein. Der Permanentmagnet oder Permanentmagnet-Werkstoff kann auch innerhalb der Verpackung oder des Behälters für das Produkt angeordnet sein. Hierbei steht dann das fertige Produkt ständig unter der Einwirkung des vom Permanentmagneten ausgehenden Magnetfeldes.

Bei der Gewinnung von Heilmittel-Extrakten kann die zur Extraktion verwendete Auszugslösung, beispielsweise Wasser, Ethanol, Isopropanol oder ein Ethanol/Wasser-Gemisch oder ein Isopropanol/Wasser-Gemisch, dem Einfluß des Magnetfeldes ausgesetzt werden.

Durch die Magnetbehandlung werden diese Auszugslösungen in eine Struktur höherer Ordnung, z. B. überstrukturiertes Wasser bzw. überstrukturiertes Ethanol, übergeführt. Dies äußert sich z. B. bei der Magnetbehandlung eines Wasser/Ethanol-Gemisches dadurch, daß eine Phasentrennung in Erscheinung tritt. Die Magnetbehandlung wurde mit einem, einen Eisenkern aufweisenden Elektromagneten durchgeführt. Die Windungszahl der Spule betrug 2000, die Spulenlänge war 5,5 cm, die wirksame Fläche betrug 12 cm$^2$, der Abstand der behandelten Flüssigkeit zur wirksamen Fläche betrug 18 mm, und das behandelte Flüssigkeitsvolumen betrug 100 ml bei 12° C. Die Spule wurde mit 0,82 A (50 Hz) beliefert. In Abhängigkeit von der Zeit der Magnetbehandlung ergab sich die aus der folgenden Tabelle ersichtliche Änderung des Mischungsverhältnisses Wasser/Ethanol, bezogen auf die Volumina.

| Zeit der Magnetbehandlung (min) | Mischungsverhältnis Wasser/Ethanol (v/v) |
|---|---|
| 0 | 0,407 |
| 2 | 0,419 |
| 5 | 0,443 |
| 15 | 0,443 |
| 30 | 0,442 |
| 60 | 0,443 |
| 180 | 0,441 |
| 240 | 0,442 |
| 300 | 0,443 |

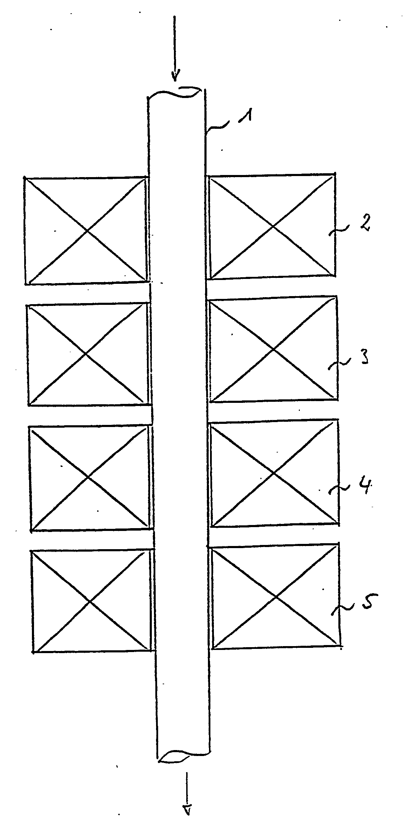

In der beiliegenden Figur ist eine Vorrichtung gezeigt, mit der eine fließfähige Substanz dem Einfluß eines Magnetfeldes in einem Durchlaufverfahren ausgesetzt werden kann. Die zu behandelnde fließfähige Substanz wird bei der dargestellten Anordnung durch ein geeignetes Kunststoffrohr 1 hindurchgeleitet, welches das von Ringmagneten 2, 3, 4 und 5 erzeugte Magnetfeld nicht abschirmt. Diese Ringmagnete bzw. zylinderförmig ausgebildeten Magnete sind so gepolt, daß die Feldlinien das Kunststoffrohr 1 axial durchsetzen. Für den Fall, daß die Pole der Magnete an den Stirnflächen liegen, erstrecken sich die magnetischen Feldlinien in axialer Richtung durch die mittleren Hohlräume, durch welche das Kunststoffrohr 1 geführt ist. Zur Förderung der zu behandelnden fließfähigen Substanz im Kunststoffrohr 1 dient eine nicht näher dargestellte Saugdruckpumpe. Hierbei kann die Substanz während eines einzigen Durchlaufs behandelt werden oder auch im Kreislauf mehrfach durch die Anordnung der Magnete 2, 3, 4 und 5 geführt werden. Diese können als Permanentmagnete oder auch als Elektromagnete ausgebildet sein. Zur Beeinflussung der fließfähigen Substanz genügen bereits relativ geringe Magnetfelder, beispielsweise mit einer mittleren magnetischen Induktion von ca. 5 x 10$^{-2}$ Tesla.

Falls erforderlich, können auch Magnetfelder mit einer höheren magnetischen Induktion von einigen, z. B. 3, Tesla zur Anwendung kommen.

Die Verweilzeit der zu behandelnden Substanz kann bei der dargestellten Anordnung von einigen Minuten bis einigen Stunden (bei einem Durchlaufverfahren) betragen. Sie kann jedoch in Abhängigkeit von der Chargengröße und der Magnetfeldstärke auch kürzer oder länger sein. Bei der Verwendung von Bädern zur Behandlung der Substanzen mit dem Magnetfeld kann die Verweilzeit auch bis zu einigen Tagen dauern.

Es wurden Untersuchungen an den in Wasser gelösten Wirkstoffen Salicylsäure und Theophyllin im Hinblick auf äußerlich anzuwendende Arzneimittel und Kosmetika durchgeführt. Bekanntlich kommt Salizylsäure in der Natur in der Spierstaude, in Sennesblättern und Kamillenblüten vor. Sie wird als Zwischenprodukt in der Pharmazie in Streupulvern, Salben, Antiseptika und auch bei der Herstellung von Pflegemitteln, Kosmetika, Riechstoffen und Sonnenschutzmitteln verwendet.

Theophyllin ist ein Purin-Derivat, das in den Blättern des Teestrauchs vorkommt. In der therapeutischen Anwendung wird es als Diuretikum, Kardiakum, Atmungsanaleptikum und Bronchalytikum verwendet.

Durch die Versuche konnte eine Steigerung der Permeationsgeschwindigkeit dieser Wirkstoffe durch Lipidmembranen nachgewiesen werden. Als Versuchsaufbau wurde eine Dialyse apparatur verwendet und die Permeationsrate durch Dodecanol-und Hexadecan-Collodium-Membranen gemessen. Es wurden Wirkstofflösungen (7,25 mmol/l) von Salicylsäure und Theophyllin gemessen. Die Dialysierfläche betrug 7,1 cm², das Donatorvolumen 300 ml und das Akzeptorvolumen 20 ml. Die Dialysierzeit betrug zwei Stunden bei 16° C, für den Flüssigkeitstransport wurde eine Saugdruckpumpe, die diskontinuierlich arbeitete, verwendet. Die Magnetbehandlung erfolgt in drei axial magnetisierten Sintermagneten mit einer mittleren magnetischen Induktion von etwa 5 x 10⁻² Tesla. In der folgenden Tabelle sind die Änderungen der Wirkstoffkonzentration im Akzeptorteil und die Steigerung der Membran bei der Permeation der Wirkstoffe angegeben.

| Membran/Wirkstoff | Wirkstoffkonzentrationen im Akzeptorteil | | Steigerung der Membranpermeation (%) |
|---|---|---|---|
| | ohne Magnet (mg/100 ml) | mit 3 Magneten (mg/ml) | |
| Dodecanolmembran Salicylsäure | 19,5 ± 1,2 | 36,0 ± 2,1 | 84,6 |
| Theophyllin | 9,4 ± 0,8 | 10,6 ± 1,0 | 12,8 |
| Hexadecanmembran Salicylsäure | 10,0 ± 1,3 | 15,0 ± 1,5 | 50,0 |
| Theophyillin | 0,34 ± 0,06 | 0,37 ± 0,07 | 8,8 |

## Ansprüche

1. Verfahren zur Behandlung von Substanzen in einem Magnetfeld, dadurch gekennzeichnet, daß die Substanzen, aus denen Kosmetika oder äußerlich anzuwendende Heilmittelextrakte oder äußerlich anzuwendende Arzneimittel hergestellt werden oder worden sind, dem Einfluß des Magnetfeldes ausgesetzt werden.

2. Verfahren nach Anspruch 1, dadurch gekennzeichnet, daß die Substanzen in einem gegebenenfalls sich wiederholenden Durchlaufverfahren durch das Magnetfeld bewegt werden.

3. Verfahren nach Anspruch 1 oder 2, dadurch gekennzeichnet, daß ein elektromagnetisches Wellenfeld verwendet wird.

4. Verfahren nach einem der Ansprüche 1 bis 3, dadurch gekennzeichnet, daß ein Magnetfeld mit einer Flußdichte von von wenigstens 10⁻² Tesla verwendet wird.

5. Verfahren nach einem der Ansprüche 1 bis 4, dadurch gekennzeichnet, daß eine Verweilzeit der Substanzen im Magnetfeld von 1 Minute bis einigen Stunden angewendet wird.

6. Verfahren nach einem der Ansprüche 1 bis 5, dadurch gekennzeichnet, daß das Extraktionsmedium bzw. eine bei der Extraktion eines Heilmittels verwendete Extraktionslösung dem Einfluß des Magnetfeldes ausgesetzt wird.

7. Verfahren nach einem der Ansprüche 1 bis 6, dadurch gekennzeichnet, daß das fertige Produkt (Kosmetikum, Heilmittel-Extrakt, Arzneimittel) dem Einfluß des Magnetfeldes ausgesetzt wird.

8. Verfahren nach Anspruch 7, dadurch gekennzeichnet, daß das abgepackte Produkt dem Einfluß des Magnetfeldes ausgesetzt wird.

9. Anordnung zur Durchführung eines der Verfahren nach einem der Ansprüche 1 bis 8, dadurch gekennzeichnet, daß an der Verpackung oder an dem Behälter für das Produkt ein Permanentmagnet oder Permanentmagnet-Werkstoff vorgesehen ist.

10. Anordnung zur Durchführung eines Verfahrens nach einem der Ansprüche 1 bis 8, dadurch gekennzeichnet, daß innerhalb der Verpackung bzw. des Behälters für das Produkt ein Permanentmagnet oder Permanentmagnet-Werkstoff vorgesehen ist.

11. Anordnung nach Anspruch 9 oder 10, dadurch gekennzeichnet, daß der Permanentmagnet als dünnes Plättchen, als Folie oder als Schicht vorliegt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.3) |
|---|---|---|---|
| X | DE-A-3 634 121 (OTTO KAUSCH) * gesamtes Dokument * | 1,2,5,7 ,8-11 | A 61 K 41/00 |
| A | | 3,4,6 | A 61 K 7/00 |
| | --- | | A 61 K 9/00 |
| X | GB-A-2 078 514 (F. DONADELLI) * Ansprüche * | 1-3,5,7 | |
| A | | 6,8-11 | |
| | --- | | |
| X | GB-A-1 264 511 (WILLY SEUSS) * Ansprüche; Seite 6, Zeile 71 - Seite 7, Zeile 17 * | 1-5,7,8 | |
| A | | 6,9-11 | |
| | --- | | |
| X | FR-A-2 588 759 (C. MOREL et al.) * gesamtes Dokument * | 9-11 | |
| | --- | | |
| X | FR-A-2 590 165 (B. BERUS) * gesamtes Dokument * | 9-11 | |
| | ----- | | |

**RECHERCHIERTE SACHGEBIETE (Int. Cl.3)**

A 61 K
A 61 J
A 45 D
B 65 D
H 05 B

**Der vorliegende Recherchenbericht wurde für alle Patentansprüche erstellt**

| Recherchenort | Abschlußdatum der Recherche | Prüfer |
|---|---|---|
| BERLIN | 13-04-1989 | SIATOU E |

EPO FORM 1503 03.82 (P0403)